# EUROPEAN PATENT APPLICATION

(11) **EP 1 726 588 A1**
(43) Date of publication of application: **29.11.2006**
(21) Application number: 05721352.2
(22) Date of filing: 16.03.2005
(51) Int. Cl.: C07D 301/14, C07D 303/04, C07D 303/34, C08G 59/24

(54) **HIGH-PURITY ALICYCLIC EPOXY COMPOUND, PROCESS FOR PRODUCING THE SAME, CURABLE EPOXY RESIN COMPOSITION, CURED ARTICLE THEREOF, AND USE**

(30) Priority: 18.03.2004 JP 2004079100
(71) Applicant: DAICEL CHEMICAL INDUSTRIES, LTD., Sakai-shi, Osaka 590-8501 (JP)
(72) Inventor: KITAO, Kyuhei, Ohtake-shi, Hiroshima 739-0651 (JP); TAKAI, Hideyuki, Ohtake-shi, Hiroshima 739-0651 (JP)
(74) Representative: Grünecker, Kinkeldey, Stockmair & Schwanhäusser Anwaltssozietät
(86) International application number: PCT/JP2005/005302
(87) International publication number: WO 2005/090325

(57) **Abstract**

The present invention provides a high-purity alicyclic epoxy compound in which an alicyclic olefin compound is epoxidized with an aliphatic percarboxylic acid having substantially no water followed by the removal of a solvent to produce an alicyclic epoxy compound represented by the general formula (I) 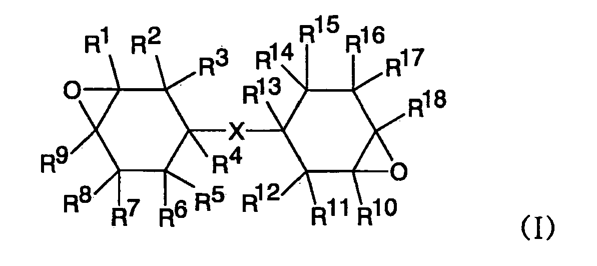 that is in turn subjected to purification by distillation to thereby the high-purity alicyclic epoxy compound wherein the concentration of high-molecular-weight components having an elution time shorter than that of the alicyclic epoxy compound in detection by a GPC analysis is 5.5% or less in terms of the area ratio, the concentration of impurities having a retention time shorter than that of the alicyclic epoxy compound in detection by a GC analysis is 19.5% or less in terms of the area ratio, the concentration of reactive intermediates is 4.5% or less in terms of the area ratio, and a color hue (APHA) is 60 or less; a process of efficiently producing the same by the use of a low-toxicity solvent; a curable resin composition using the same; a cured product; and applications.

## Description

### Technical Field

The present invention relates to a high-purity alicyclic epoxy compound obtained by oxidizing an alicyclic olefin compound with an aliphatic percarboxylic acid containing substantially no water followed by purification; a process for the production of the same; a curable epoxy resin composition; a cured product thereof; and a variety of applications of the high-purity alicyclic epoxy compound. In particular, the present invention relates to a high-purity alicyclic epoxy compound reduced in the concentration of impurities or reactive intermediates and improved in a color hue; a process for the production of the compound through purification by distillation; a curable epoxy resin composition containing the high-purity alicyclic epoxy compounds; and a cured product thereof; and a variety of applications of the high-purity alicyclic epoxy compound. The high-purity alicyclic epoxy compound is useful in applications that require heat resistance or transparency, including transparent materials for encapsulation, transparent films, transparent sheets, insulating materials between layers, coatings, inks, adhesives, sealants, stabilizers, insulating materials, and display materials such as liquid crystals.

### Background Art

Various kinds of diepoxy compounds each having two alicyclic skeletons in the molecule are currently commercially available, including CEL-2021P (3,4-epoxycyclohexylmethyl-3',4'-epoxycyclohexanecarboxylate), and CEL-3000 (1,2,8,9-diepoxylimonene), CEL-2081 (3,4-epoxycyclohexylmethanol at one terminal of an ε-caprolacton oligomer and 3,4-epoxycyclohexanecalboxylic acid at another end thereof, respectively linked by an ester bond) [all of which are manufactured by Daicel Chemical Industries Co., Ltd.]. The above-described CEL-3000 has an epoxy group of lower reactivity because of having a methyl group in carbon constituting the epoxy group, than those having no methyl group. Alternatively, the CEL-2021P and CEL-2081 have hydrolyzability because of having an ester bond in the molecule. Therefore, when they are used under high temperature and humidity conditions or such a condition that a strong acid occurs, a cured product may be reduced in the physical property. Thus, there has been a demand for an epoxy compound having an alicyclic skeleton with no ester bond in the molecule.

Meanwhile, JP 48-29899 A describes a compound represented by the general formula (I) where X is -CH₂- and R¹ to R¹⁸ each are a hydrogen atom is synthesized and then used for curing reaction with an acid anhydride to provide a cured product improved in the physical property as compared to conventional alicyclic epoxy compounds. However, the epoxy compound is synthesized using a perbenzoic acid and therefore has difficulty in industrial usage. Alternatively, in JP 58-172387 A, a percarboxylic acid is synthesized from a hydrogen peroxide, an acid catalyst, and an organic acid and then extracted with an organic solvent, followed by epoxidation using this extract. Consequently, there are a large amount of wastes and complicated procedures in addition to time-consuming operations. Besides, because this percarboxylic acid contains, but in a very small quantity, a hydrogen peroxide and an acid catalyst in addition to water, the percarboxylic acid may be rendered unstable and reduced in concentration in a short time at the reaction step and the extraction step for producing the percarboxylic acid. Moreover, the reduced concentration brings the inside of a reactor into a risky condition on account of generating oxygen at the same time. Furthermore, at the epoxidation reaction step and the purification step for a resulting epoxide, side reaction of the epoxide is likely to occur, resulting in reduction in the amount of collected products. In addition, this method could be industrially disadvantageous because the production device is contaminated with products in side-reaction.

Moreover, the above-described JP 58-172387 A discloses perpropionic acid, perbutyric acid, and perisobutyric acid as an available percarboxylic acid. However, as compared to a peracetic acid preferably used in the present invention, these percarboxylic acids have large molecular weights and are therefore reduced in the ability as an oxidizing agent and productivity per unit weight. Acetic acid and acetaldehyde are more inexpensive materials and more advantageous in terms of costs than propionic acid and butyric acid. Moreover, acetic acid has a lower boiling point than that of propionic acid or butyric acid and thus advantageous in terms of energy when collected and distilled.

In JP 2002-275169 A, a compound having two alicyclic olefin skeletons with no intramolecular ester bond is epoxidized with peracetic acid obtained by the air oxidation of acetaldehyde to synthesize the above-described alicyclic epoxy compound (I). However, in the synthesis of the epoxy compound (I), the removal of a solvent is merely carried out. Therefore, there remain high-molecular-weight parts having an elution time shorter than that of the alicyclic epoxy compound (I) detected by a GPC analysis and impurities or reactive intermediates having a retention time shorter than that of the alicyclic epoxy compound(I) in detection by gas chromatography, thereby giving the epoxy compound (I) that is not sufficient in a color hue (APHA) and is not sufficient to be used in transparent materials that require heat resistance including display materials such as liquid crystals.

An object of the present invention is to provide a process of efficiently producing a high-purity alicyclic epoxy compound by the use of a low-toxicity solvent, in which an alicyclic olefin compound is epoxidized with an aliphatic percarboxylic acid having substantially no water followed by purification to thereby produce the high-purity alicyclic epoxy compound without an ester bond that is reduced in the concentration of high-molecular-weight components each having an elution time shorter than that of the alicyclic epoxy compound (I) in detection by a GPC analysis and in the concentration of impurities or reactive intermediates each having a retention time shorter than that of the alicyclic epoxy compound (I) in detection by a gas chromatography and that is improved in a color hue, and to provide a cured product enhanced especially in transparency and heat resistance.

According to the present invention, a high-purity alicyclic epoxy compound represented by the general formula (I) can be efficiently produced by the use of a low-toxicity solvent, in which an alicyclic olefin compound is epoxidized with an aliphatic percarboxylic acid having substantially no water followed through purification to thereby produce the high-purity alicyclic epoxy compound without ester bond that is reduced in the concentration of high-molecular-weight components each having an elution time shorter than that of the alicyclic epoxy compound represented by the general formula (I) in detection by a GPC analysis and in the concentration of impurities or reactive intermediates each having a retention time shorter than that of the alicyclic epoxy compound represented by the general formula (I) in detection by gas chromatography and that is improved in a color hue.

### Disclosure of the Invention

The inventors of the present invention have attained the above object and completed the present invention by finding that the epoxidation of a compound having two alicyclic olefin skeletons is conducted by the use of an aliphatic percarboxylic acid obtained by the air oxidization of an aliphatic aldehyde followed through purification by distillation.

That is, a first invention provides a high-purity alicyclic epoxy compound represented by the following general formula (I) : 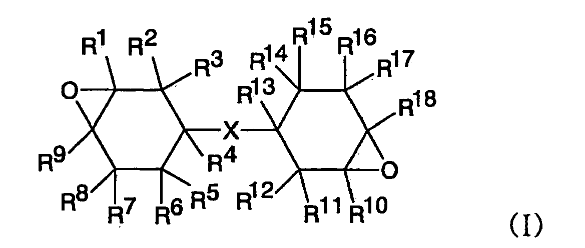 (wherein X is a divalent group selected from the group consisting of an oxygen atom, a sulfur atom, -SO-, -SO₂-, -CH₂-, -C(CH₃)₂-, -CBr₂-, -C (CBr₃)₂-, and -C (CF₃)₂-, and R¹ to R¹⁸ may be identical or different from each other and are each a hydrogen atom, a halogen atom, a hydrocarbon group that may contain an oxygen atom or halogen atom, or an alkoxy group that may have a substituent),
in which the concentration of high-molecular-weight components each having an elution time shorter than that of the alicyclic epoxy compound represented by the general formula (I) in detection by gel permeation chromatography (hereinafter, GPC) is 5.5% or less with respect to the sum total of all of detected peak areas in terms of a peak area ratio per elution time.

A second invention provides a high-purity alicyclic epoxy compound according to the first invention, in which the concentration of impurities each having a retention time shorter than that of the alicyclic epoxy compound represented by the above general formula (I) in detection by gas chromatography is 19.5% or less with respect to the sum total of all of detected peak areas in terms of the peak area ratio per retention time.

A third invention provides a high-purity alicyclic epoxy compound according to the first or second invention, in which the concentration of reactive intermediate compounds each represented by the following general formula (III): 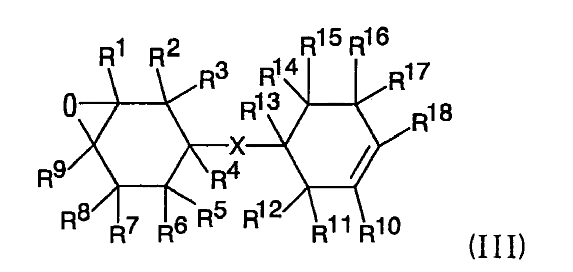 (wherein X is a divalent group selected from the group consisting of an oxygen atom, a sulfur atom, -SO-, -SO₂-, -CH₂-, -C (CH₃)₂-, -CBr₂-, -C (CBr₃) ₂-, and -C (CF₃) ₂-, and R¹ to R¹⁸ may be identical or different from each other and are each a hydrogen atom, a halogen atom, a hydrocarbon group that may contain an oxygen atom or halogen atom, or an alkoxy group that may have a substituent),
in detection by gas chromatography is 4.5% or less with respect to the sum total of all of detected peak areas in terms of the peak area ratio per retention time.

A fourth invention provides a high-purity alicyclic epoxy compound according to the first to third inventions, in which a color hue (APHA) is 60 or less.

A fifth invention provides a high-purity alicyclic epoxy compound according to any one of the first to fourth inventions, in which the alicyclic epoxy compound is produced by epoxidizing, with an aliphatic percarboxylic acid having substantially no water, an alicyclic olefin compound represented by the following general formula (II): 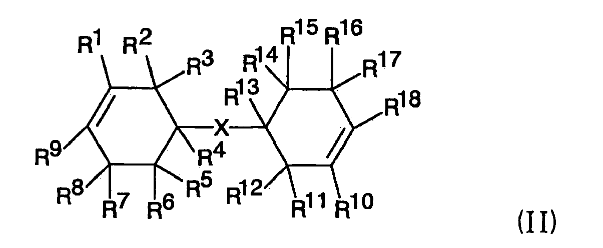 (wherein X is a divalent group selected from the group consisting of an oxygen atom, a sulfur atom, -SO-, -SO₂-, -CH₂-, -C(CH₃)₂-, -CBr₂-, -C(CBr₃)₂-, and -C(CF₃)₂-, and R¹ to R¹⁸ may be identical or different from each other and are each a hydrogen atom, a halogen atom, a hydrocarbon group that may contain an oxygen atom or halogen atom, or an alkoxy group that may have a substituent),
which is 95.0% or more with respect to the sum total of all of detected peak areas in terms of the peak area ratio determined by gas chromatography.

A sixth invention provides a high-purity alicyclic epoxy compound according to the fifth invention, in which the alicyclic epoxy compound is obtained by epoxidation followed by removal of a solvent and purification by distillation.

A seventh invention provides a process for the production of a high-purity alicyclic epoxy compound, including:
epoxidizing an alicyclic olefin compound represented by the following general formula (II) 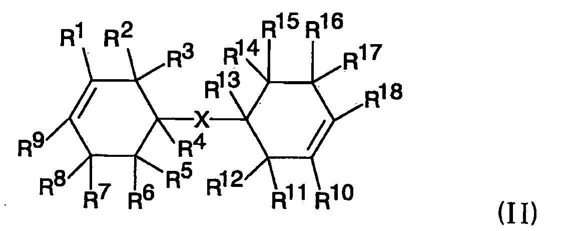 with an aliphatic percarboxylic acid having substantially no water followed by removal of a solvent to produce an alicyclic epoxy compound represented by the general formula (I) 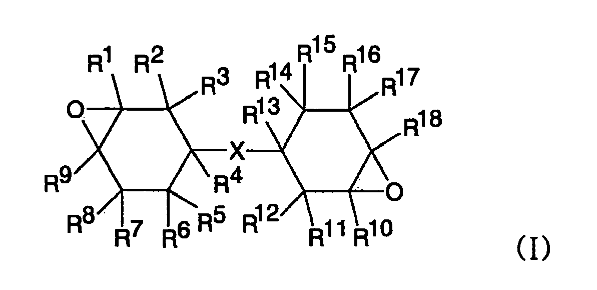 (in the formulas (I) and (II), X is a divalent group selected from the group consisting of an oxygen atom, a sulfur atom, -SO-, -SO₂-, -CH₂-, -C(CH₃)₂-, -CBr₂-, -C(CBr₃)₂-, and -C(CF₃)₂-, and R¹ to R¹⁸ may be identical or different from each other and are each a hydrogen atom, a halogen atom, a hydrocarbon group that may contain an oxygen atom or halogen atom, or an alkoxy group that may have a substituent); and
   subjecting the produced alicyclic epoxy compound to purification by distillation to thereby produce the high-purity alicyclic epoxy compound in which the concentration of high-molecular-weight components each having an elution time shorter than that of the alicyclic epoxy compound in detection by a GPC analysis is 5.5% or less with respect to a sum total of all of detected peak areas in terms of a peak area ratio per elution time.

An eighth invention provides the process for the production of a high-purity alicyclic epoxy compound according to the seventh invention, in which the concentration of impurities each having a retention time shorter than that of the alicyclic epoxy compound represented by the above general formula (I) in detection by gas chromatography is 19.5% or less with respect to the sum total of all of detected peak areas in terms of the peak area ratio per retention time.

A ninth invention provides the process for the production of a high-purity alicyclic epoxy compound according to the seventh or eighth invention, in which the concentration of reactive intermediate compounds each represented by the above general formula (III) in detection by gas chromatography is 4.5% or less with respect to the sum total of all of detected peak areas in terms of the peak area ratio per retention time.

A tenth invention provides the process for the production of a high-purity alicyclic epoxy compound according to any one of the seventh to ninth inventions, in which a color hue (APHA) is 60 or less.

An eleventh invention provides the process for the production of a high-purity alicyclic epoxy compound according to any one of the seventh to tenth inventions, in which the aliphatic percarboxylic acid is obtained by oxidation of a corresponding aldehyde with oxygen.

A twelfth invention provides the process for the production of a high-purity alicyclic epoxy compound according to any one of the seventh to eleventh inventions, in which a water content in the aliphatic percarboxylic acid is 0.8% by weight or less.

A thirteenth invention provides the process for the production of a high-purity alicyclic epoxy compound according to any one of the seventh to twelfth inventions, in which the aliphatic percarboxylic acid is a peracetic acid.

A fourteenth invention provides the process for the production of a high-purity alicyclic epoxy compound according to any one of the seventh to thirteenth inventions, in which the purification by distillation is carried out at a heating temperature ranging from 100 to 350°C and at a pressure of 50 to 0.01 Torr.

A fifteenth invention provides the process for the production of a high-purity alicyclic epoxy compound according to any one of the eighth to fourteenth inventions, in which the aliphatic percarboxylic acid is an ethyl acetate solution.

A sixteenth invention provides a photo-curable and/or heat-curable epoxy resin composition, characterized by including: the epoxy compound according to any one of the first to seventh inventions; an epoxy group-containing compound optionally added; and a curing agent or a curing catalyst.

A seventeenth invention provides a cured product that is obtained by curing the curable epoxy resin composition according to the sixteenth invention.

An eighteenth invention provides a transparent material for encapsulation, characterized by including the high-purity alicyclic epoxy compound according to anyone of the first to seventh inventions.

A nineteenth invention provides an adhesive, characterized by including the high-purity alicyclic epoxy compound according to any one of the first to seventh inventions.

A twentieth invention provides the cured product according to the eighteenth invention, in which the cured product is at least one selected from a transparent film, transparent sheet, an insulating material between layers, a coated film, and a paint film.

### Brief Description of the Drawings

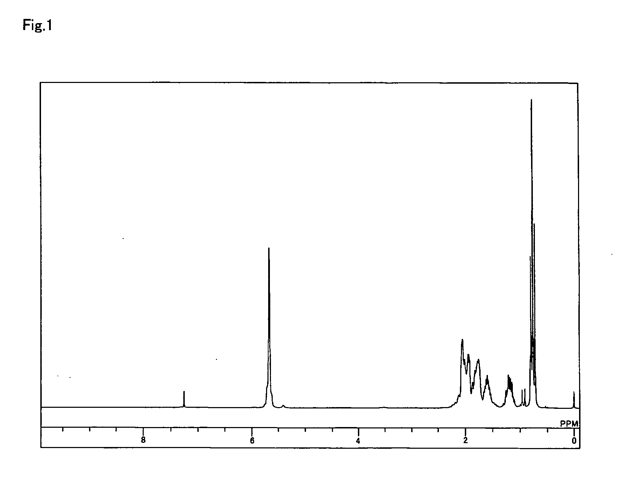

Fig. 1 is a ¹H-NMR chart of 2,2-bis (3',4'-cyclohexenyl) propane as a raw material.

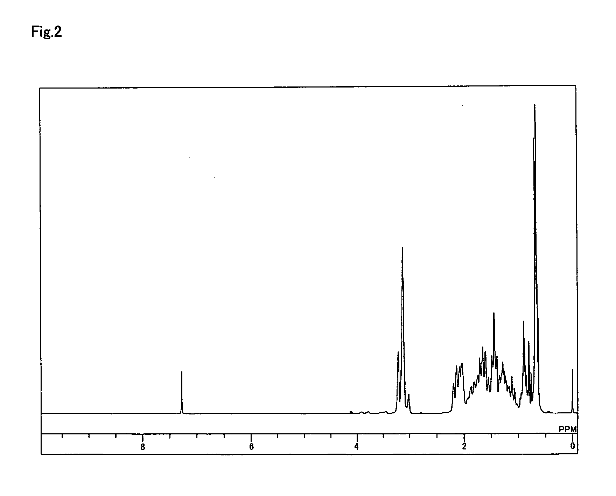

Fig. 2 is a ¹H-NMR chart of an alicyclic epoxy compound (IA) obtained in Example 1.

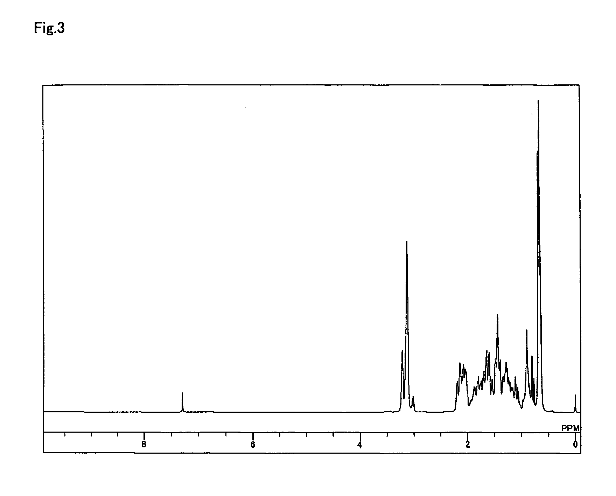

Fig. 3 is a ¹H-NMR chart of an alicyclic epoxy compound (IA) obtained in Example 4.

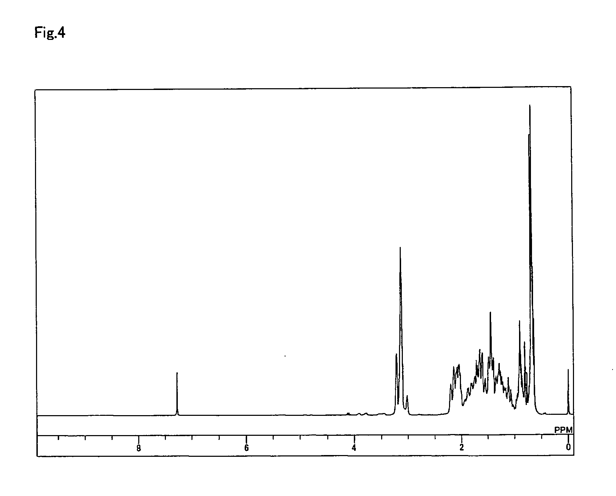

Fig. 4 is a ¹H-NMR chart of an alicyclic epoxy compound (IA) obtained in Comparative Example 1.

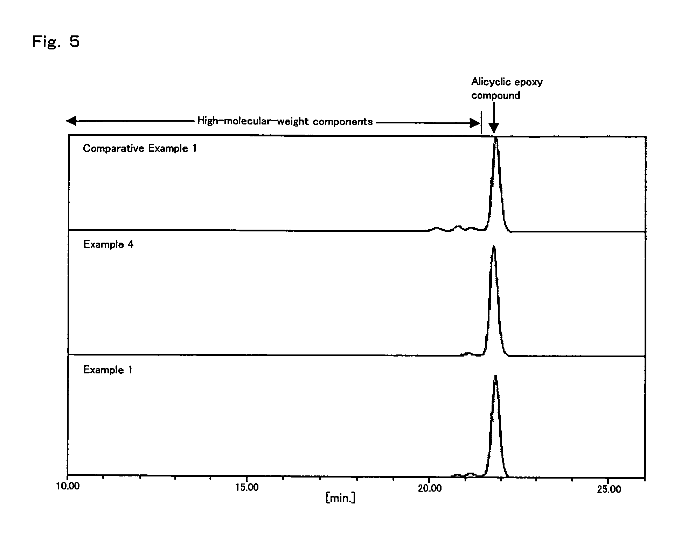

Fig. 5 is a GPC chart of the alicyclic epoxy compounds (IA) obtained in Example 1, Example 4, and Comparative Example 1.

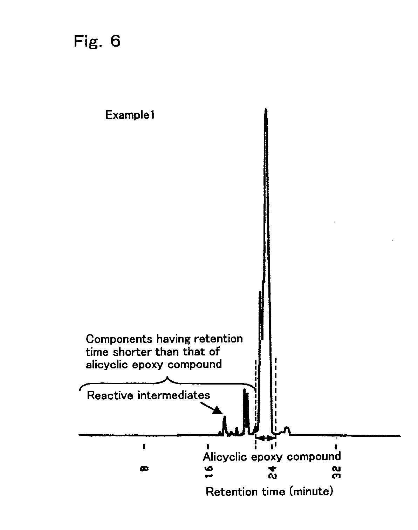

Fig. 6 is a gas chromatograph chart of the alicyclic epoxy compound (IA) obtained in Example 1.

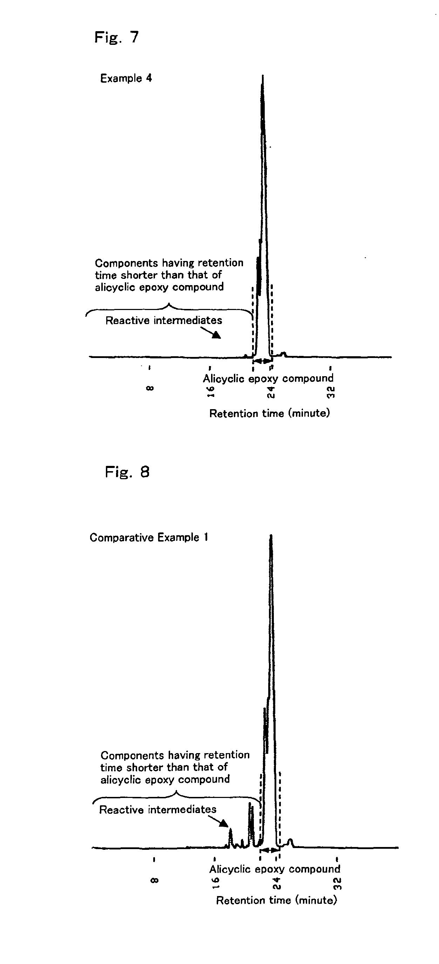

Fig. 7 is a gas chromatograph chart of the alicyclic epoxy compound (IA) obtained in Example 4.
Fig. 8 is a gas chromatograph chart of the alicyclic epoxy compound (IA) obtained in Comparative Example 1.

### Best Mode for carrying out the Invention

Hereinafter, the embodiments of the present invention will be described.

A high-purity alicyclic epoxy compound without an ester bond represented by the above general formula (I) of the present invention is produced by epoxidizing an alicyclic olefin compound represented by the general formula (II) with an aliphatic percarboxylic acid having substantially no water followed through purification by distillation.

The alicyclic olefin compound (II) used as a raw material is generally synthesized by the dehydration reaction of a corresponding compound having a hydroxyl group. In a process for the production of the alicyclic olefin compound (II) , the alicyclic olefin compound can be synthesized from, for example, a compound having a cyclohexanol structure, as described in JP-A 48-29899, JP-A 58-172387, and JP-A 2000-169399. As obvious from the general formula (II) , the obtained alicyclic olefin compound (II) preferably has double bonds at the positions 3 and 4 with respect to a substituent X, and the compound having a hydroxyl group given as a raw material of the alicyclic olefin compound (II) preferably has a hydroxyl group at the position 4 with respect to the substituent X. For the same purpose described above, the present invention is especially effective on the dehydration reaction of a compound having at least two cyclohexane rings bound with hydroxyl groups in the molecule, which is given as an example of the above-described compound.

Examples of the compounds having at least two cyclohexane rings bound with hydroxyl groups in the molecule include hydrobisphenol, dicyclohexanolmethane, bis(dimethylcyclohexanol)methane, 1,2-bis(cyclohexanol)ethane, 1,3-bis(cyclohexanol)propane, 1,4-bis(cyclohexanol)butane, 1,5-bis(cyclohexanol)pentane, 1,6-bis(cyclohexanol)hexane, 2,2-bis(cyclohexanol)propane, bis(cyclohexanol)phenylmethane, α,α-bis(4-hydroxycyclohexyl)-4-(4-hydroxy-α,α-dimethylcyclohex ylmethyl)-ethylbenzene, 3,3-bis(cyclohexanol)pentane, 5,5-bis(cyclohexanol)heptane, dodecahydrofluorenediol, tris(cyclohexanol)methane, tris(cyclohexanol)ethane, 1,3,3-tris(cyclohexanol)butane, tetrakis(cyclohexanol)ethane, 2,2-bis[4,4'-bis(cyclohexanol)cyclohexyl]propane, bisphenol C hydride (C: cyclohexane), hydropolyphenol, and mixtures thereof.

An aliphatic percarboxylic acid having substantially no water is employed as an epoxidizing agent that can be used in the epoxidation of the double bond in the alicyclic olefin compound (II). This is because an epoxidation reaction performed in the presence of water facilitates the ring-opening reaction of an epoxy group, thereby reducing the yield of the epoxy compound. Thus, the aliphatic percarboxylic acid contains substantially no water, more particularly 0.8% by weight or less of water, preferably 0.6% by weight or less of water. The aliphatic percarboxylic acid having substantially no water according to the present invention refers to peracetic acid or the like produced by the air oxidization of acetaldehyde or the like. For example, the peracetic acid is produced by a process described in DE 1418465 A or JP-A 54-3006. According to this process, aliphatic percarboxylic acids can be continuously synthesized in large amounts and in high concentrations and can be thus obtained at a substantially low cost, as compared to the case that an aliphatic percarboxylic acid is synthesized from hydrogen peroxide and extracted with a solvent to produce the aliphatic percarboxylic acid.

Available aliphatic percarboxylic acids are performic acid, peracetic acid, pertrifluoroacetic acid, and the like. Of those, especially, the peracetic acid is a preferred epoxidizing agent in terms of industrially inexpensive availability and high stability. The amount of the aliphatic percarboxylic acid as an epoxidizing agent is not particularly limited. The optimum amount in each situation is determined depending on variables such as each used epoxidizing agent, the desired degree of epoxidation, and each used substance subjected to epoxidation. If a compound having a large number of epoxy groups in one molecule is desired, it is preferred that the number of moles of an epoxidizing agent to be added be equal to or greater than that of an olefin group. However, an epoxidizing agent exceeding 2-fold mole with respect to an olefin group is usually disadvantageous in light of economy and side reaction described below, and peracetic acid is preferably added in the range of 1-fold to 1.5-fold mole with respect to use amount of an olefin group.

An epoxidation reaction is carried out with or without an inert solvent and by regulating reaction temperatures, depending on devices or the physical properties of raw materials. The inert solvent can be used for the purpose of reducing the viscosity of raw materials, stabilizing an epoxidizing agent by dilution, and so on. In the case of peracetic acid, an aromatic compound, an ester, or the like can be used as the solvent. Especially preferred solvents are hexane, cyclohexane, toluene, ethyl acetate, and methyl acetate. The amount of a solvent to be used ranges from 0.1-fold to 10-fold weight, preferably from 0.3-fold to 3-fold weight, with respect to the weight of olefin.

If a solvent has a weight of less than 0.1-fold weight, the amount of an adduct of acetic acid is increased in epoxidation. If a solvent has a weight greater than 10-fold weight, epoxidation takes much time and requires a large-size device, thereby reducing productivity.

The available range of reaction temperatures is determined depending on the reactivity of a epoxidizing agent to be used. In general, the temperature is 0°C to 100°C both inclusive. Concerning peracetic acid as a preferred epoxidizing agent, the temperature preferably ranges from 20°C to 70°C. At or below 20°C, the reaction is rendered slow, while at 70°C, the degradation of the peracetic acid occurs. For a reaction, no particular operation is necessary and, for example, a mixture may be stirred for 1 to 5 hours. The isolation of a resulting epoxy compound can be carried out by an appropriate method, for example, a method involving precipitating the epoxy compound with a poor solvent, a method involving adding the epoxy compound in hot water while stirring to distill and remove a solvent, or a method involving directly removing a solvent.

However, in an alicyclic epoxy compound (I) obtained by any one of those methods, which had an insufficient color hue, there remained high-molecular-weight components detected by a GPC analysis and impurities or reactive intermediates each having a retention time shorter than that of the alicyclic epoxy compound (I) in detection by gas chromatography, as described in Background Art.

The high-molecular-weight components detected by a GPC analysis include a polymerized material of the alicyclic epoxy compound (I), and the monoadduct or polyadduct of a carboxylic acid secondarily produced from an epoxidizing agent to the alicyclic epoxy compound (I).

Examples of the monoadduct of a carboxylic acid secondarily produced from an epoxidizing agent to the alicyclic epoxy compound (I) include 1-acetoxy-2-hydroxy-1',2'-epoxy-4,4'-methylenedicyclohexane, 1-acetoxy-2-hydroxy-2,2',6,6'-tetramethyl-1',2'-epoxy-4,4'-met hylenedicyclohexane, 1-acetoxy-2-hydroxy-1',2'-epoxy-4,4'-ethylenedicyclohexane, 1-acetoxy-2-hydroxy-1',2'-epoxy-4,4'-(propane-1,3-diyl)dicyclo hexane, 1-acetoxy-2-hydroxy-1',2'-epoxy-4,4'-(butane-1,4-diyl)dicycloh exane, 1-acetoxy-2-hydroxy-1',2'-epoxy-4,4'-(pentane-1,5-diyl)dicyclo hexane, 1-acetoxy-2-hydroxy-1',2'-epoxy-4,4'-(hexane-1,6-diyl)dicycloh exane, 2-(3-hydroxy-4-acetoxycyclohexyl)-2-(3,4-epoxycyclohexyl)propa ne, 1-acetoxy-2-hydroxy-1',2'-epoxy-4,4'-(phenylmethylene)dicycloh exane, α,α-bis(3,4-epoxycyclohexyl)-4-((3-hydroxy-4-acetoxy-α,α-dimet hylcyclohexyl)methyl)ethylbenzene, 3-(3-hydroxy-4-acetoxycyclohexyl)-3-(3,4-epoxycyclohexyl)penta ne, 3-(3-hydroxy-4-acetoxycyclohexyl)-3-(3,4-epoxycyclohexyl)hepta ne, 2-hydroxy-3-acetoxy-6,7-epoxydodecahydrofluorene, (3-hydroxy-4-acetoxycyclohexyl)-bis(3,4-epoxycyclohexyl)methan e, 1-(3-hydroxy-4-acetoxycyclohexyl)-2,2-bis(3,4-epoxycyclohexyl) ethane, 1-(3-hydroxy-4-acetoxycyclohexyl)-3,3-bis(3,4-epoxycyclohexyl) ethane, 1-(3-hydroxy-4-acetoxycyclohexyl)-1,2,2-tris(3,4-epoxycyclohex yl)ethane, 2-(4-(3-hydroxy-4-acetoxycyclohexyl)cyclohexyl)-2-(4-(',4-epox ycyclohexyl)cyclohexyl)propane, and 2-(3-hydroxy-3-methyl-4-acetoxycyclohexyl)-2-(3-methyl-3,4-epo xycyclohexyl)propane.

Examples of the polyadduct of a carboxylic acid secondarily produced from an epoxidizing agent to the alicyclic epoxy compound (I) include bis(3-hydroxy-4-acetoxycyclohexyl)methane, bis (3-hydroxy-3,5-dimethyl-4-acetoxycyclohexyl)methane, 1,2-bis(3-hydroxy-4-acetoxycyclohexyl)ethane, 1,3-bis(3-hydroxy-4-acetoxycyclohexyl)propane, 1,4-bis(3-hydroxy-4-acetoxycyclohexyl)butane, 1,5-bis(3-hydroxy-4-acetoxycyclohexyl)pentane, 1,6-bis(3-hydroxy-4-acetoxycyclohexyl)hexane, 2,2-bis(3-hydroxy-4-acetoxycyclohexyl)propane, bis(3-hydroxy-4-acetoxycyclohexyl)phenylmethane, α,α-bis(3-hydroxy-4-acetoxycyclohexyl)-4-((3-hydroxy-4-acetoxy -α,α-dimethylcyclohexyl)methyl)ethylbenzene, 3,3-bis(3-hydroxy-4-acetoxycyclohexyl)pentane, 3,3-bis(3-hydroxy-4-acetoxycyclohexyl)heptane, 2,6-dihydroxy-3,7-diacetoxydodecahydrofluorene, tris(3-hydroxy-4-acetoxycyclohexyl)methane, 1,1,2-tris(3-hydroxy-4-acetoxycyclohexyl)ethane, 1,1,3-tris(3-hydroxy-4-acetoxycyclohexyl)butane, tetrakis(3-hydroxy-4-acetoxycyclohexyl)ethane, 2,2-bis[4,4'-bis(3-hydroxy-4-acetoxycyclohexyl)cyclohexyl]prop ane, and 2,2-bis(3-hydroxy-3-methyl-4-acetoxycyclohexyl)propane.

The impurities each having a retention time shorter than that of the alicyclic epoxy compound (I) in detection by a gas chromatography include: a solvent used in epoxidation such as hexane, cyclohexane, toluene, ethyl acetate, or methyl acetate; a carboxylic acid secondarily produced from an epoxidizing agent such as formic acid, acetic acid, or trifluoroacetic acid; a compound derived from a solvent used for synthesizing the alicyclic olefin compound (II) such as naphthalene or tetramethylbenzene; an olefin monool compound and an epoxidized material of the olefin monool compound secondarily produced in the synthesis of the alicyclic olefin compound (II); a monoolefin compound and an epoxidized material of the monoolefin compound as impurities secondarily produced in the synthesis of the alicyclic olefin compound (II) ; and a monoadduct of a carboxylic acid secondarily produced from an epoxidizing agent to the epoxidized material of the monoolefin compound.

Examples of the olefin monool compounds secondarily produced in the synthesis of the alicyclic olefin compound (II) include 4-(3-cyclohexenyl)methylcyclohexanol, 3,5-dimethyl-4-(3,5-dimethyl-3-cyclohexenyl)methylcyclohexanol, 4-(2-(3-cyclohexenyl)ethyl)cyclohexanol, 4-(3-(3-cyclohexenyl)propyl)cyclohexanol, 4-(4-(3-cyclohexyenyl)butyl)cyclohexanol, 4-(4-(3-cyclohexenyl)pentyl)cyclohexanol, 4-(5-(3-cyclohexenyl)hexyl)cyclohexanol, 4-((1-(3-cyclohexenyl)-1-methyl)ethyl)cyclohexanol, 4-(3-cyclohexenylphenylmethyl)cyclohexanol, α,α-bis(3-cyclohexenyl)-4-(4-hydroxy-α,α-dimethylcyclohexylmet hyl)ethylbenzene, 4-(1-(3-cyclohexenyl)-1-ethyl)propylcyclohexanol, 4-(1-(3-cyclohexenyl)-1-ethyl)pentylcyclohexanol, 2-hydroxy-[1,2,3,4,5,8,4a,4b,8a,8b]decahydrofluorene, 4-bis(3-cyclohexenyl)methylcyclohexanol, 4-bis(3-cyclohexenyl)ethylcyclohexanol, 4-bis(3-cyclohexenyl)butylcyclohexanol, 4-(1,2,2-tris(3-cyclohexenyl))ethylcyclohexanol, 4-(4-(4-(1-(3-cyclohexenyl)-1-methyl)ethylcyclohexyl)methylcyc lohexyl)cyclohexanol, and 2-methyl-4-(1-(3-methyl-3-cyclohexenyl)-1-methyl)ethylcyclohex anol.

Examples of the epoxidized material of the olefin monool compound include 4-(3,4-epoxycyclohexylmethyl)cyclohexanol, 3,5-dimethyl-4-(3,5-dimethyl-3,4-epoxycyclohexylmethyl)cyclohe xanol, 4-(2-(3,4-epoxycyclohexyl)ethyl)cyclohexanol, 4-(3-(3,4-epoxycyclohexyl)propyl)cyclohexanol, 4-(4-(3,4-epoxycyclohexyl)butyl)cyclohexanol, 4-(5-(3,4-epoxycyclohexyl)pentyl)cyclohexanol, 4-(6-(3,4-epoxycyclohexyl)hexyl)cyclohexanol, 4-(1-(3,4-epoxycyclohexyl)-1-methylethyl)cyclohexanol, 4-((3,4-epoxycyclohexylphenyl)methyl)cyclohexanol, α,α-bis(3,4-epoxycyclohexyl)-4-(4-hydroxy-α,α-dimethylcyclohex ylmethyl)ethylbenzene, 4-(1-(3,4-epoxycyclohexyl)-1-ethylpropyl)cyclohexanol, 4-(1-(3,4-epoxycyclohexyl)-1-ethylpentyl)cyclohexanol, 2-hydroxy-[1,2,3,4,5,8,4a,4b,8a,8b]dodecahydrofluorene, 4-bis(3,4-epoxycyclohexyl)methylcyclohexanol, 4-bis(3,4-epoxycyclohexyl)ethylcyclohexanol, 4-bis(3,4-epoxycyclohexyl)butylcyclohexanol, 4-(1,2,2-tris(3,4-epoxycyclohexyl))ethylcyclohexanol, 4-(4-(1-(4-(3,4-epoxycyclohexyl)cyclohexyl)-1-methyl)ethylcycl ohexyl)cyclohexanol, and 2-methyl-4-(1-(3-methyl-3,4-epoxycyclohexyl)-1-metyl)ethylcycl ohexanol.

Examples of the monoolefin compound as an impurity secondarily produced in the synthesis of the alicyclic olefin compound (II) include 4-(cyclohexylmethyl)cyclohexene, 2,6-dimethyl-4-(3,5-dimethylcyclohexylmethyl)cyclohexene, 1-(3-cyclohexenyl)-2-cyclohexylethane, 1-(3-cyclohexenyl)-3-cyclohexylpropane, 1-(3-cyclohexenyl)-4-cyclohexylbutane, 1-(3-cyclohexenyl)-5-cyclohexylpentane, 1-(3-cyclohexenyl)-6-cyclohexylhexane, 2-(3-cyclohexenyl)-2-cyclohexylpropane, 3-cyclohexenylcyclohexylphenylmethane, α,α-dicyclohexyl-4-(α,α-dimethyl-3-cyclohexenylmethyl)ethylben zene, 3-(3-cyclohexenyl)-3-cyclohexylpentane, 3-(3-cyclohexenyl)-3-cyclohexylheptane, [1,2,3,4,5,8,4a,4b,8a,8b]decahydrofluorene, 4-(dicyclohexylmethyl)cyclohexene, 1-(3-cyclohexenyl)-2,2-dicyclohexylethane, 1-(3-cyclohexenyl)-3,3-dicyclohexylbutane, 1-(3-cyclohexenyl)-1,2,2-tricyclohexylethane, 2-(4-(3-cyclohexenyl)cyclohexyl)-2-bicyclohexylpropane, and 2-(3-methyl-3-cyclohexenyl)-2-(3-methylcyclohexyl)propane.

Examples of the epoxidized material of the monoolefin compound as an impurity secondarily produced in the synthesis of the alicyclic olefin compound (II) include 1,2-epoxy-4,4'-methylene-dicyclohexane, 1,2-epoxy-2,2',6,6'-tetramethyl-4,4'-methylenedicyclohexane, 1-(3,4-epoxycyclohexyl)-2-cyclohexylethane, 1-(3,4-epoxycyclohexyl)-3-cyclohexylpropane, 1-(3,4-epoxycyclohexyl)-4-cyclohexylbutane, 1-(3,4-epoxycyclohexyl)-5-cyclohexylpentane, 1-(3,4-epoxycyclohexyl)-6-cyclohexylhexane, 2-(3,4-epoxycyclohexyl)-2-cyclohexylpropane, (3,4-epoxycyclohexyl)-2-cyclohexylphenylmethane, α,α-dicyclohexyl-4-(α,α-dimethyl-3,4-epoxycyclohexylmethyl)eth ylbenzene, 3-(3,4-epoxycyclohexyl)-3-cyclohexylpentane, 3-(3,4-epoxycyclohexyl)-3-cyclohexylheptane, 2,3-epoxy-[1,2,3,3,4,5,8,4a,4b,8a,8b]decahydrofluorene, 4-(bis(3,4-epoxycyclohexyl)methyl)cyclohexene, 1-(3,4-epoxycyclohexyl)-2,2-bicyclohexylethane, 1-(3,4-epoxycyclohexyl)-3,3-bicyclohexylbutane, 1-(3,4-epoxycyclohexyl)-1,2,2-triscyclohexylethane, 2-(4-(3,4-epoxycyclohexyl)cyclohexyl)-2-bicyclohexylpropane, and 2-(3-methyl-3,4-epoxycyclohexyl)-2-(3-methylcyclohexyl)propane

Examples of the carboxylic acid monoadduct secondarily produced from an epoxidizing agent to the epoxidized product of the monoolefin compounds as an impurity secondarily produced in the synthesis of alicyclic olefin compound (II) include 1-acetoxy-2-hydroxy-4-(cyclohexylmethyl)cyclohexane, 1-acetoxy-2-hydroxy-2,6-dimethyl-4-(3,5-dimethylcyclohexylmeth yl)cyclohexane, 1-(3-hydroxy-4-acetoxycyclohexyl)-2-cyclohexylethane, 1-(3-hydroxy-4-acetoxycyclohexyl)-3-cyclohexylpropane, 1-(3-hydroxy-4-acetoxycyclohexyl)-4-cyclohexylbutane, 1-(3-hydroxy-4-acetoxycyclohexyl)-5-cyclohexylpentane, 1-(3-hydroxy-4-acetoxycyclohexyl)-6-cyclohexylhexane, 2-(3-hydroxy-4-acetoxycyclohexyl)-2-cyclohexylpropane, (3-hydroxy-4-acetoxycyclohexyl)cyclohexylphenylmethane, α,α-dicyclohexyl-4-(α,α-dimethyl-3-hydroxy-4-acetoxycyclohexyl methyl)ethylbenzene, 3-(3-hydroxy-4-acetoxycyclohexyl)-3-cyclohexylpentane, 3-(3-hydroxy-4-acetoxycyclohexyl)-3-cyclohexylheptane, 2-hydroxy-3-acetoxy-[1,2,3,4,5,8,4a,4b,8a,8b]decahydrofluorene, 4-(3-hydroxy-4-acetoxycyclohexyl)cyclohexene, 1-(3-hydroxy-4-acetoxycyclohexyl)-2,2-biscyclohexylethane, 1-(3-hydroxy-4-acetoxycyclohexyl)-3,3-biscyclohexylbutane, 1-(3-hydroxy-4-acetoxycyclohexyl)-1,2,2,-triscyclohexylethane, 2-(4-(3-hydroxy-4-acetoxycyclohexyl)cyclohexyl)-2-bicyclopropa ne, and 2-(3-methyl-3-hydroxy-4-acetoxycyclohexyl)-2-(3-methylcyclohex yl)propane.

Each of the reactive intermediates detected by gas chromatography is a reactive intermediate in which one double bond in the alicyclic olefin compound represented by the above general formula (II) is epoxidized with a monoepoxy/monoolefin compound represented by the above general formula (III), and is converted to the alicyclic epoxy compound represented by the above general formula (I) by the additional epoxidation of the remaining double bond.

The inventors of the present invention have made a study of the synthesis and purification of the alicyclic epoxy compound (I) by the above-described method and, as a result, found that compounds derived from the high-molecular-weight components, the reactive intermediates, and the impurities in raw materials can be removed through purification by distillation, thereby providing an alicyclic epoxy compound improved in a color hue and a cured product improved in transparency.

A process for the production of the high-purity alicyclic epoxy compound represented by the general formula (I) of the present invention is a process of distilling and purifying the alicyclic epoxy compound obtained at the above-described step, in which compounds derived from high-molecular-weight components, reactive intermediates, and impurities in raw materials can be removed, thereby providing an alicyclic epoxy compound enhanced in a color hue. In the high-purity alicyclic epoxy compound represented by the general formula (I) of the present invention, the concentration of high-molecular-weight components detected by a GPC analysis is 5.5%, preferably 4.1% or less, more preferably 2.5% or less, with respect to the sum total of all of detected peak areas; the concentration of impurities each having a retention time shorter than that of the alicyclic epoxy compound represented by the above general formula (I) in detection by gas chromatography is 19.5% or less, preferably 16.4% or less, more preferably 13.0% or less, with respect to the sum total of all of detected peak areas in terms of the peak area ratio per retention time; the concentration of reactive intermediates is 4.5% or less, preferably 3.5% or less, more preferably 0.1% or less, with respect to the sum total of all of detected peak areas in terms of the peak area ratio per retention time; and a color hue (APHA) is 60 or less, preferably 40 or less, more preferably 20 or less.

A purification process can utilize, alone or in combination, general methods including single stage batch distillation and distillation with a thin film evaporator such as WEF (Wiped Film Evaporator) or FFE (Falling Film Evaporator), a molecular still, or a distillation column. Of those, the thin film evaporator where an epoxy compound is not susceptible to heat is preferably used. Distillation with the thin film evaporator or the like is performed on such a condition that a pressure is 50 to 0.01 Torr, preferably 20 to 0.03 Torr, more preferably 10 to 0.05 Torr; and a heating temperature is 100 to 350°C, preferably 120 to 330°C, more preferably 150 to 300°C.

The alicyclic epoxy compound (I) that has been distilled and purified is a high-purity alicyclic epoxy compound that is reduced in the concentration of high-molecular-weight components detected by a GPC analysis and in the concentration of compounds derived from reactive intermediates and impurities in raw materials detected by gas chromatography and that is improved in a color hue, and can be therefore subjected to homopolymerization, copolymerization, or additional reaction with other compounds, thereby producing a variety of coatings, inks, adhesives, sealants, moldings or intermediates for other applications using these.

A curable epoxy resin composition of the present invention is characterized by including: the high-purity alicyclic epoxy compound represented by the general formula (I) of the present invention; an epoxy group-containing compound optionally added; and a curing agent or a curing catalyst.

The curing agent or the curing catalyst is a cationic polymerization initiator or an acid anhydride that generates cationic species through light or heat. Because of having the photocationic polymerization initiator and/or the thermal cationic polymerization initiator, or the acid anhydride as the curing agent to be an essential component, the curable epoxy resin composition of the present invention can be cured/polymerized through light or heat.

Available photocationic polymerization initiators are, for example, sulfonium salt base, iodonium salt base, diazonium salt base, and allene-ion complex base compounds, including: sulfonium salt base compounds such as UVACURE1590 and UVACURE1591 (from Daicel UCB Co., Ltd.), DAICAT11 (from Daicel Chemical industries, Ltd.), CD-1011 (from Sartomer Co.), and SI-60L, SI-80L, and SI-100L (from Sanshin Chemical Industries, Ltd.); iodonium salt base compounds such as DAICAT12 (from Daicel Chemical Industries, Ltd.) and CD-1012 (from Sartomer Co.) ; and diazonium salt base compounds such as SP-150 and SP-170 (from Asahi Denka Co., Ltd.). Of those photocationic polymerization initiators, the above-described SI-60L, SI-80L, and SI-100L can also generate cations by heating.

In addition, a silanol base cationic catalyst such as triphenylsilanol or an aluminum chelate base catalyst such as aluminum tris(acetylacetone) can be used as the thermal cationic polymerization initiator.

In the present invention, the above-described cationic polymerization initiator on the order of 0.01 to 20 parts by weight, preferably 0.1 to 5 parts by weight, more preferably 0.1 to 3 parts by weight is suitably blended with 100 parts by weight of the total epoxy compounds. 0.01 part by weight or less of the initiator is not preferable because such an amount significantly reduces the heat-curability of the epoxy resin composition. In contrast, whereas the initiator beyond 20 part by weight is uneconomical because no effect by increasing is shown and the physical properties of a cured product reduce.

Additionally, in the present invention, an acid anhydride can be also used as the curing agent. Preferably, the acid anhydride is an acid anhydride with carbon atoms on the order of 4 to 25, preferably 8 to 20 having, in the molecule, one or two aliphatic rings or aromatic rings as well as one or two acid anhydride groups, such as tetrahydrophthalic anhydride, methyltetrahydrophthalic anhydride, methylhexahydrophthalic anhydride, hexahydrophthalic anhydride, methylhimic anhydride, pyromellitic dianhydride, benzophenonetetracarboxylic dianhydride, 3,3',4,4'-biphenyltetracarboxylic dianhydride, bis(3,4-dicarboxyphenyl) ether dianhydride, bis(3,4-dicarboxyphenyl) methane dianhydride, and 2,2'-bis(3,4-dicarboxyphenyl) propane dianhydride.

In this case, an acid anhydride in which the content of a compound having a carboxyl group (COOH group) is 0.5% by weight or less (i.e., 0-0.5% by weight) , particularly 0. 4% by weight or less (i.e., 0-0.4% by weight) is used. A content of a carboxyl group greater than 0.5% by weight is not preferred, because crystallization may occur. In this case, for the same reason, the content of a carboxyl group (COOH group) is preferably 0.3% by weight or less (i.e., 0-0.3% by weight), particularly 0.25% by weight or less (i.e., 0-0.25% by weight), relative to the acid anhydride as a curing agent.

It is noted that, in the amount of an acid anhydride to be blended, the ratio of an acid anhydride group in the curing agent is preferably in the range of 0.3 to 0.7 mole relative to 1 mole of an epoxy group in the epoxy resin. If the ratio is less than 0.3 mole, the curing property is insufficient. In contrast, if the ratio is above 0.7 mole, unreacted acid anhydrides may remain, causing reduction in glass-transition temperatures. More preferably, the ratio is in the range of 0.4 to 0.6 mole.

Additionally, examples of a curing accelerator that can be used for curing include, but is not limited to: an amidine compound such as 1,8-diazabicyclo(5,4,0) undecene (DBU); an organic phosphorous compound such as triphenylphosphine, tetraphenylphosphonium, or tetraphenylborate; and an imidazole compound such as 2-methylimidazole. Those curing accelerators may be used alone or mixed with each other. The amount of a curing accelerator to be blended is preferably in the range of 0.4 to 20 parts by weight relative to 100 parts by weight of the total amount of the alicyclic epoxy compound represented by the above general formula (I) and the polymerization initiator. If the curing accelerator less than 0.4 part by weight is blended, the curing property may not be sufficient in heat molding. On the other hand, the amount beyond 20 parts by weight is not preferred, because curing may be so fast that failure in loading occurs owing to lower fluidity in molding.

In the curable epoxy resin composition of the present invention, the other epoxy group-containing compound to be added as required together with the high-purity alicyclic epoxy compound, the curing agent, and the curing accelerator is not particularly limited in terms of its molecular structure, molecular weight, and so on as long as the compound has at least two epoxy groups in the molecule and an epoxy resin used for sealing semiconductors can be used as it is. Specific examples thereof include biphenyl type epoxy resins, bisphenol type epoxy resins, stilbene type epoxy resins, phenol novolak type epoxy resins, cresol novolak type epoxy resins, naphthalene type epoxy resins, triphenolmethane type epoxy resins, alkyl-modified triphenolmethane type epoxy resins, triazine nucleus-containing epoxy resins, and dicyclopentadiene-modified phenol type epoxy resins. Each of those epoxy resins may be used alone, or two or more kinds of them may be mixed before use.

In addition to above examples, the examples also include: glycidyl ester type epoxy resins such as dimer acid glycidyl ester and triglycidyl ester; glycidyl amine type epoxy resins such as tetraglycidyl amino diphenylmethane, triglycidyl-p-aminophenol, triglycidyl-p-aminophenol, tetraglycidyl methaxylenediamine, and tetraglycidyl bisaminomethylcyclohexane; heterocyclic epoxy resins such as triglycidyl isocyanurate; trifunctional epoxy resins such as phloroglucinol triglycidyl ether, trihydroxy biphenyl triglycidyl ether, trihydroxy phenylmethane triglycidyl ether, glycerin triglycidyl ether, 2-[4-(2,3-epoxypropoxy)phenyl]-2-[4-[1,1-bis[4-(2,3-epoxypropo xy)phenyl]ethyl]phenyl]propane, and 1,3-bis[4-[1-[4-(2,3-epoxypropoxy)phenyl]-1-[4-[1-[4-(2,3-epox ypropoxy)phenyl]-1-methylethyl]phenyl]ethyl]phenoxy]-2-propano 1; tetrafunctional epoxy resins such as tetrahydroxyphenylethane teteraglycidyl ether, teteraglycidyl benzophenone, bisresorcinol teteraglycidyl ether, and tetraglycidoxybiphenyl; and alicyclic epoxy resins such as 3,4-epoxycyclohexenylmethyl 3',4'-epoxycyclohexenyl carboxylate (Celloxide 2021P available from Daicel Chemical Industries) , limonen diepoxide (Celloxide 3000 available from Daicel Chemical Industries), ε-caprolactone-modified 3,4-epoxycyclohexyenylmethyl-3',4'-epoxycyclohexyl carboxylate (Celloxide 2081 available from Daicel Chemical Industries), bis(3,4-epoxycyclohexylmethyl)adipate (such as ERL4227 available from Union Carbide), epoxidized 3-cyclohexene-1,2-dicarboxylic acid bis 3-cyclohexenylmethyl ester and ε-caprolactone adducts thereof (GT300 series such as "Epolead GT301" available from Daicel Chemical Industries), and epoxidized butane tetracarboxylic acid tetrakis-3-cyclohexenyl methylester and ε-caprolactone adducts thereof (GT400 series such as "Epolead GT401" available from by Daicel Chemical Industries).

The content of other epoxy group-containing compounds is 0 to 90 parts by weight, preferably 15 to 85 parts by weight, more preferably 20 to 80 parts by weight, relative to 100 parts by weight of the total amount of the above-described high-purity alicyclic epoxy compound and the epoxy group-containing compounds.

The above-described other epoxy group-containing compounds less than 15 parts by weight is disadvantageous in terms of a cost. In contrast, the content greater than 90 parts by weight reduces the effect of the high-purity alicyclic epoxy compound of the present invention.

In order to mix the respective above-described components, after sufficiently mixed with a generally utilized apparatus, for example, a mixer such as a blender, the components are further dissolved and kneaded with a heat roll, a kneader, and so on and are then cooled, followed by pulverization to give a molding material. Moreover, for sealing electronic parts such as semiconductor elements to produce a semiconductor device, sealing is performed by a molding method such as transfer molding, compression molding, or injection molding.

The curable epoxy resin composition of the present invention is cured at 30 to 240°C, preferably 35 to 180°C, more preferably 35 to 60°C for 30 to 300 minutes, preferably 45 to 240 minutes, more preferably 60 to 120 minutes.

If the temperature and the time for curing are under the lower limits of the above-mentioned ranges, the curing is rendered insufficient. In contrast, if they are above the upper limits of the above-mentioned ranges, the decomposition of the resin component may occur. Thus, both cases are not preferred. The curing condition, which depends on various conditions, can be adjusted appropriately so that the curing time is rendered shorter with increases in the curing temperature, and the curing time is rendered longer with decreases in the curing temperature. Typically, it is preferred that following primary curing (curing temperature: 30-240°C, preferably 35-180°C, more preferably 35-60°C; time: 30-300 minutes, preferably 45-240 minutes, more preferably 60-120 minutes), secondary curing (curing temperature: 60-240°C, preferably 90-200°C, more preferably 120-200°C; curing time: 30-180 minutes, preferably 45-150 minutes, more preferably 60-120 minutes) be performed in order not to cause inadequate curing.

The curable epoxy resin composition of the present invention can be also cured by irradiation with light such as ultraviolet rays or active energy beams, for example, electron beams.

Used as a light source in performing irradiation of ultraviolet rays is, for example, a high-pressure mercury lamp, a super high-pressure mercury lamp, a carbon arc lamp, a xenon lamp, or a metal halide lamp. Although irradiation time differs depending on the kind of the light source, a distance between the light source and the applied surface, and other conditions, the time is 50 or 60 seconds at most and normally a few seconds. After irradiation of ultraviolet rays, curing can be completely attained by heating, if necessary. In the case of irradiation by electron beams, electron beams having energy in the range of 50 to 1,000 KeV is used and 2-5 Mrad dose of irradiation is preferable. Typically, an irradiation source having a lamp output on the order of 80 to 300 W/cm is utilized.

Since the high-purity alicyclic epoxy compound as an essential resin component in the curable epoxy resin composition of the present invention has low viscosity, its curable epoxy resin composition also features low viscosity and excellent processability. In addition, the alicyclic epoxy compound dose not volatilize in temperature regions less than 100°C and thus does not affect working environment.

Examples of the final use of the high-purity alicyclic epoxy compound represented by the general formula (I) of the present invention include transparent materials for encapsulation, transparent films, transparent sheets, insulating materials between layers, acid scavengers, furniture coatings, decorative coatings, coatings for beverage cans and the other cans, adhesives, undercoatings for automobiles, sealers, finishings, inks for textual information or image information, sealants for electronic parts, photoresists suitable for manufacturing printing plates or printing circuit boards, casting print rolls, formulations for molded material or molded products made of formulations for forming sheets, solvents, flame retardants, and intermediates for producing other compounds useful in various final uses including pharmaceuticals and medical implements. The high-purity alicyclic epoxy compound represented by the general formula (I) of the present invention can impart heat resistance, transparency, and favorable dielectric properties as features of a resin using a compound having an alicyclic skeleton to a cured product, and can be employed without any problem in transparent materials for the display purpose such as liquid crystals that require heat resistance.

### Examples

Although the present invention will be more fully described hereinafter with reference to Examples and Applied Examples, the present invention is not intended to be limited to them. Unless otherwise specified, "%" used in Examples refers to "% by weight".

### <GPC analysis>

In pretreatment, 0.04 g of an alicyclic epoxy compound (I) is dissolved in 2 g of tetrahydrofuran (THF) and filtered with a filter having a pore size of 0.50 µm [DISMIC13JP050AN manufactured by Toyo Roshi Kaisha, Ltd.]. The resulting THF solution of the alicyclic epoxy compound (I) is analyzed by a GPC to give the rate of a peak area for each of the components as the concentration of each of the components. The respective concentrations of components eluted earlier than the alicyclic epoxy compound (I) are summed to determine the concentration of high-molecular-weight components.
Apparatus: HLC-8220GPC [manufactured by TOSO CORPORATION]
Detector: differential refractometer (RI detector)
Precolumn: TSKGUARDCOLUMN SUPER HZ-L 4.6 mm × 20 mm
Column: sample side TSK-GEL SUPER HZM-N 4.6 mm × 150 mm × 4
: reference side TSK-GEL SUPER HZM-N 6.0 mm × 150 mm × 1
+ TSK-GEL SUPER H-RC 6.0 mm × 150 mm
Thermostat temperature: 40°C
Moving phase: THF
Flow amount of moving phase: 0.35 ml/min
Sample amount: 10µl
Data sampling interval: 10 min to 26 min after sample injection

### <Gas chromatography>

An alicyclic epoxy compound (I) is directly analyzed by gas chromatography without pretreatment. The rate of a peak area of each of the components with respect to the sum total of all of detected peak areas is given as the concentration of each of the components.
Apparatus:GC14-B type (manufactured by Shimadzu Corporation)
Column: Thermon 3000/5% Shincarbon A 2.6 mm x 3 m
Nitrogen flow: 40 ml/min
Air pressure: 60 kPa·s/cm²
Hydrogen pressure: 60 kPa·s/cm²

Thermostat temperature: retain 60°C for 2 min , then elevate up to 250°C by 10°C /min, and retain for 19 min
Injection port temperature: 250°C
Detector side temperature: 250°C
Detector: FID
RANGE: 10^3
Injection amount: alicyclic epoxy compound (I) 1 µl
Data sampling interval: up to 40 min from just after sample injection
Data processor: C-R5A (manufactured by Shimadzu Corporation)
Min. Area: 100
SLOPE: 700
DRIFT: AUTO

### (Production Example 1)

To a 300 ml stainless reactor equipped with an air inlet, a perforated plate for gas dispersion, and a cooling jacket, a 10% acetaldehyde-ethyl acetate solution containing cobalt acetate was added at a rate of 114 kg/hour while compressed air was injected into the reactor, to carry out a reaction at 45°C. The reaction solution contained peracetic acid of 10.1%, acetoaldehyde monoperacetate of 2.2%, and acetic acid of 2.0%. This solution was placed together with sodium polyphosphate in a distillation column and condensed to give a peracetic acid solution. This peracetic acid solution contained peracetic acid of 29.1% and water of 0.47%.

### [Example 1]

To a 3 L jacketed flask equipped with a stirrer, a cooling tube, a thermometer, and a nitrogen-injecting tube, 36 g of water, 12.0 g of sodium hydrogensulfate, 500 g of isopropylidene-4,4'-dicyclohexanol (manufactured by ALDRICH), and 500 g of Solvesso 150 as a solvent (manufactured by Exxon Chemical) were added and subjected to a dehydration reaction at 100°C. The reaction was terminated at the completion of distillation of water. When the reaction solution was analyzed by gas chromatography, 2,2-bis(3',4'-cyclohexenyl) propane was generated in an yield of 96%. After the obtained reaction solution was washed in 500 ml of ion exchanged water using a separatory funnel, the organic layer was distilled under reduced pressure to give 387.0 g of 2,2-bis(3',4'-cyclohexenyl) propane in a colorless and transparent liquid state at a purity of 96.1%. To a 1 L jacketed flask as described above, 100 g of this 2,2-bis(3',4'-cyclohexenyl) propane and 300 g of ethyl acetate were added. Then, 307.2 g of an ethyl acetate solution (peracetic acid concentration: 29.1%, watercontent: 0.47%) of peracetic acid having substantially no water, which was obtained in Production Example 1, was added dropwise to the flask over approximately 2 hours to increase the temperature inside the reaction system to 30°C, with nitrogen injected to the gas phase portion. After the completion of dropping of the peracetic acid, the solution was aged at 30°C for 3 hours and the reaction was then terminated. After the post-reaction solution was further washed in water at 30°C and low-boiling fractions were removed at 70°C/20 Torr, the solution was distilled in a WFE-type thin film evaporator at a heating temperature of 180°C and a pressure of 4 Torr to give 69.6 g of a product having an alicyclic epoxy compound (IA) as a main ingredient. The alicyclic epoxy compound (IA) is an alicyclic epoxy compound represented by the general formula (I) in which X is -C(CH₃)₂- and R¹ to R¹⁸ each are a hydrogen atom (which was similarly given in the description below).

With regard to the properties of the obtained product, the concentration of oxirane oxygen was 12.4% (theoretical value: 13.5%), the viscosity is 1,890 cP (25°C), and the color hue (APHA) was 15. In ¹H-NMR, a peak around δ4.5 to 5 ppm that originated from the inner double bond almost disappeared and the formation of proton peak around δ2.9 to 3.1 ppm that originated from the epoxy group was observed. In this case, the concentration of high-molecular-weight components each having an elution time shorter than that of the alicyclic epoxy compound (IA) in detection by GPC analysis was 5.2% in terms of the area ratio, the concentration of impurities each having a retention time shorter than that of the alicyclic epoxy compound (IA) in detection by gas chromatography was 12.1% in terms of the area ratio, and the concentration of reactive intermediates was 2.3% in terms of the area ratio.

### [Example 2]

To a 3 L jacketed flask equipped with a stirrer, a cooling tube, a thermometer, and a nitrogen-injecting tube, 300 g of 4,4'-dicyclohexanolmethane, 600 g of toluene, and 3 g of paratoluenesulfonic acid were added and subjected to a dehydration reaction at 110°C. The reaction was terminated at the completion of distillation of water. When the reaction solution was analyzed by gas chromatography, di(3,4-cyclohexenyl) methane was generated in a yield of 96%. After the obtained reaction solution was washed in 500 ml of ion exchanged water using a separatory funnel, the organic layer was distilled under reduced pressure to give 269 g of di(3,4-cyclohexenyl) methane in a colorless and transparent liquid state at a purity of 96.8%.

To a 1 L jacketed flask as described above, 100 g of this di (3, 4-cyclohexenyl) methane and 200 g of ethyl acetate were added. Then, 276.2 g of an ethyl acetate solution (peracetic acid concentration: 29.1%) of peracetic acid having substantially no water, which was obtained in Production Example 1, was added dropwise to the flask over approximately 3 hours to increase the temperature inside the reaction system to 25°C, with nitrogen injected to the gas phase portion. After the completion of dropping of the peracetic acid, the solution was aged at 30°C for 4 hours and the reaction was then terminated. After the post-reaction solution was further washed in water at 30°C and low-boiling fractions were removed at 70°C/30 Torr, the solution was distilled in a WFE-type thin film evaporator at a heating temperature of 180°C and a pressure of 5 Torr to give 74.5 g of a product having an alicyclic epoxy compound (IB) as a main component. The alicyclic epoxy compound (IB) is an alicyclic epoxy compound represented by the general formula (I) in which X is -CH₂- and R¹ to R¹⁸ each are a hydrogen atom (which was similarly given in the description below). With regard to the properties of the obtained product, the concentration of oxirane oxygen was 14.5%, the viscosity was 2,190 cP (25°C), and the color hue (APHA) was 20. In ¹H-NMR, a peak around δ4.5 to 5 ppm that originated from the double bond almost disappeared and the formation of proton peak around δ2.9 to 3.3 ppm that originated from the epoxy group was observed. In this case, the concentration of high-molecular-weight components each having an elution time shorter than that of the alicyclic epoxy compound (IB) in detection by GPC analysis was 3.7% in terms of the area ratio, the concentration of impurities each having a retention time shorter than that of the alicyclic epoxy compound (IB) in detection by gas chromatography was 7.1% in terms of the area ratio, and the concentration of reactive intermediates was 2.8% in terms of the area ratio.

### [Example 3]

The reaction was carried out as in Example 2 to give 330 g of di(3,4-cyclohexenyl) sulfone at a purity of 95.2%, except that 400 g of hydrogenated bisphenolsulfone (i.e., 4,4'-dicyclohexanolsulfone) and 500 g of Solvesso 150 as a solvent (manufactured by Exxon Chemical) were used.

To a 1 L jacketed flask as described above, 100 g of this reaction product and 300 g of ethyl acetate were added. Then, 242.7 g of an ethyl acetate solution (peracetic acid concentration: 29.1%) of peracetic acid having substantially no water, which was obtained in Production Example 1, was added dropwise to the flask over approximately 2 hours to increase the temperature inside the reaction system to 40°C, with nitrogen injected to the gas phase portion. After the completion of dropping of the peracetic acid, the solution was aged at 40°C for 4 hours and the reaction was then terminated. After the crude solution was further washed in water at 30°C and low-boiling fractions were removed at 70°C/30 Torr, the solution was simply distilled at a heating temperature of 180°C and a pressure of 1 Torr to give 71.0 g of a product having an alicyclic epoxy compound (IC) as a main ingredient. The alicyclic epoxy compound (IC) is an alicyclic epoxy compound represented by the general formula (I) in which X is -SO₂- and R¹ to R¹⁸ each are a hydrogen atom (which was similarly given in the description below).

With regard to the properties of the obtained product, the concentration of oxirane oxygen was 11.9%, and the viscosity was 3, 600 cP (25°C). In ¹H-NMR, a peak around δ4.5 to 5 ppm that originated from the double bond almost disappeared and the formation of proton peak around δ2.9 to 3.1 ppm that originated from the epoxy group was observed. In this case, the concentration of high-molecular-weight components each having an elution time shorter than that of the alicyclic epoxy compound (IC) in detection by GPC analysis was 3.5% in terms of the area ratio, the concentration of impurities each having a retention time shorter than that of the alicyclic epoxy compound (IC) in detection by gas chromatography was 8.7% in terms of the area ratio, and the concentration of reactive intermediates was 2.4% in terms of the area ratio.

### [Example 4]

To a 3 L jacketed flask equipped with a stirrer, a cooling tube, a thermometer, and a nitrogen-injecting tube, 72 g of water, 24.0 g of sodium hydrogensulfate, 1,000 g of isopropylidene-4,4'-dicyclohexanol (manufactured by ALDRICH), and 1,000 g of Solvesso 150 as a solvent (manufactured by Exxon Chemical) were added and subjected to a dehydration reaction at 100°C.

The reaction was terminated at the completion of distillation of water. When the reaction solution was analyzed by gas chromatography, 2,2-bis(3',4'-cyclohexenyl) propane was generated in a yield of 96%. After the obtained reaction solution was washed in 500 ml of ion exchanged water using a separatory funnel, the organic layer was distilled under reduced pressure to give 774.0 g of 2,2-bis (3',4'-cyclohexenyl) propane in a colorless and transparent liquid state at a purity of 95.7%.

To a 1 L jacketed flask as described above, 300 g of this 2, 2-bis (3',4'-cyclohexenyl) propane and 600 g of ethyl acetate were added. Then, 321.6 g of an ethyl acetate solution (peracetic acid concentration: 29.1%, water content: 0.47%) of peracetic acid having substantially no water, which was obtained in Production Example 1, was added dropwise to the flask over approximately 2 hours to increase the temperature inside the reaction system to 30°C, with nitrogen injected to the gas phase portion. After the completion of dropping of the peracetic acid, the solution was aged at 30°C for 3 hours and the reaction was then terminated. After the post-reaction solution was further washed in water at 30°C and low-boiling fractions were removed at 70°C/20 Torr, the solution was distilled and purified in a 5-stages Older Shaw distillation column at a heating temperature of 236°C and a pressure of 2 Torr to give 199.7 g of a product having the alicyclic epoxy compound (IA) as a main component.

With regard to the properties of the obtained product, the concentration of oxirane oxygen was 13.4%, the viscosity was 1,940 cP (25°C), and the color hue (APHA) was 10. In ¹H-NMR, a peak around δ4.5 to 5 ppm that originated from the inner double bond almost disappeared and the formation of proton peak around δ2.9 to 3.1 ppm that originated from the epoxy group was observed. In this case, the concentration of high-molecular-weight components each having an elution time shorter than that of the alicyclic epoxy compound (IA) in detection by GPC analysis was 2.0% in terms of the area ratio, the concentration of impurities each having a retention time shorter than that of the alicyclic epoxy compound (IA) in detection by gas chromatography was 1.2% in terms of the area ratio, and the concentration of reactive intermediates was 0.0% in terms of the area ratio.

### [Example 5]

To a 3 L jacketed flask equipped with a stirrer, a cooling tube, a thermometer, and a nitrogen-injecting tube, 72 g of water, 24.0 g of sodium hydrogensulfate, 1,000 g of isopropylidene-4,4'-dicyclohexanol (manufactured by ALDRICH), and 1, 000 g of Solvesso 150 as a solvent (manufactured by Exxon Chemical) were added and subjected to a dehydration reaction at 100°C.

The reaction was terminated at the completion of distillation of water. When the reaction solution was analyzed by gas chromatography, 2,2-bis(3',4'-cyclohexenyl) propane was generated in a yield of 96%. After the obtained reaction solution was washed in 500 ml of ion exchanged water using a separatory funnel, the organic layer was distilled under reduced pressure to give 774.0 g of 2,2-bis (3',4'-cyclohexenyl) propane in a colorless and transparent liquid state at a purity of 95.7%.

To a 1 L jacketed flask as described above, 300 g of this 2, 2-bis (3' , 4' -cyclohexenyl) propane and 600 g of ethyl acetate were added. Then, 321.6 g of an ethyl acetate solution (peracetic acid concentration: 29.1%, water content: 0.47%) of peracetic acid having substantially no water, which was obtained in Production Example 1, was added dropwise to the flask over approximately 2 hours to become the temperature inside the reaction system to 30°C, with nitrogen injected to the gas phase portion. After the completion of dropping of the peracetic acid, the solution was aged at 30°C for 3 hours and the reaction was then terminated. After the post-reaction solution was further washed in water at 30°C and low-boiling fractions were removed at 70°C/20 Torr, the low-boiling fractions were removed in a 10-stages Older Shaw distillation column at a heating temperature of 210°C and a pressure of 2 Torr. The resultant bottom was distilled in a WFE-type thin film evaporator at a temperature of 180°C and a pressure of 5 Torr to give 174.7 g of a product having the alicyclic epoxy compound (IA) as a main ingredient. With regard to the properties of the obtained product, the concentration of oxirane oxygen was 13.1%, the viscosity was 2,240 cP (25°C), and the color hue (APHA) was 15. In ¹H-NMR, a peak around δ4.5 to 5 ppm that originated from the inner double bond almost disappeared and the formation of proton peak around δ2.9 to 3.1 ppm that originated from the epoxy group was observed. In this case, the concentration of high-molecular-weight components each having an elution time shorter than that of the alicyclic epoxy compound (IA) in detection by GPC analysis was 2.3% in terms of the area ratio; the concentration of impurities each having a retention time shorter than that of the alicyclic epoxy compound (IA) in detection by gas chromatography was 1.1% in terms of the area ratio, and the concentration of reactive intermediates was 0.0% in terms of the area ratio.

### [Comparative Example 1]

To the 3 L jacketed flask used in Example 1, 100 g of the 2,2-bis(3',4'-cyclohexenyl) propane synthesized in Example 1 and 300 g of ethyl acetate were added. Then, 307.2 g of an ethyl acetate solution (peracetic acid concentration:29.1%, water content:0.47%) of peracetic acid having substantially no water, which was obtained in Production Example 1, was added dropwise to the flask over approximately 2 hours to become the temperature inside the reaction system to 30°C, with nitrogen injected to the gas phase portion. After the completion of dropping of the peracetic acid, the solution was aged at 30°C for 3 hours and the reaction was then terminated. The post-reaction solution was further washed in water at 30°C and low-boiling fractions were removed at 70°C/20 Torr to give 99.4 g of a product having the alicyclic epoxy compound (IA). With regard to the properties of the obtained product, the concentration of oxirane oxygen was 11.3%, the viscosity was 3,550 cP (25°C), and the color hue (APHA) was 90. In ¹H-NMR, a peak around δ4.5 to 5 ppm that originated from the inner double bond almost disappeared and the formation of proton peak around δ2.9 to 3.1 ppm that originated from the epoxy group was observed. In this case, the concentration of high-molecular-weight components each having an elution time shorter than that of the alicyclic epoxy compound (IA) in detection by GPC analysis was 12.5% in terms of the area ratio, the concentration of impurities each having a retention time shorter than that of the alicyclic epoxy compound (IA) in detection by gas chromatography was 11.9% in terms of the area ratio, and the concentration of reactive intermediates was 2.0% in terms of the area ratio.

### [Comparative Example 2]

To the 3 L jacketed flask used in Example 1, 100 g of the di(3,4-cyclohexenyl) methane synthesized in Example 2 and 200 g of ethyl acetate were added. Then, 276.2 g of an ethyl acetate solution (peracetic acid concentration: 29.1%) of peracetic acid having substantially no water, which was obtained in Production Example 1, was added dropwise to the flask over approximately 3 hours to become the temperature inside the reaction system to 25°C, with nitrogen injected to the gas phase portion. After the completion of dropping of the peracetic acid, the solution was aged at 30°C for 4 hours and the reaction was then terminated. The post-reaction solution was further washed in water at 30°C and low-boiling fractions were removed at 70°C/30 Torr to give 106.4 g of a product having the alicyclic epoxy compound (IB). With regard to the properties of the obtained product, the concentration of oxirane oxygen was 13.8%, the viscosity was 2,590 cP (25°C), and the color hue (APHA) was 110. In ¹H-NMR, a peak around δ4.5 to 5 ppm that originated from the double bond almost disappeared and the formation of proton peak around δ2.9 to 3.3 ppm that originated from the epoxy group was observed. In this case, the concentration of high-molecular-weight components each having an elution time shorter than that of the alicyclic epoxy compound (IB) detected by a GPC analysis was 14.7% in terms of the area ratio, the concentration of impurities each having a retention time shorter than that of the alicyclic epoxy compound (IB) detected by a gas chromatography was 7.9% in terms of the area ratio, and the concentration of reactive intermediates was 2.7% in terms of the area ratio.

### [Comparative Example 3]

To the 3 L jacketed flask used in Example 1, 100 g of the di (3,4-cyclohexenyl) sulfone synthesized in Example 3 and 300 g of ethyl acetate were added. Then, 242.7 g of an ethyl acetate solution (peracetic acid concentration: 29.1%) of peracetic acid was added dropwise to the flask over approximately 2 hours to become the temperature inside the reaction system to 40°C, with nitrogen injected to the gas phase portion. After the completion of dropping of the peracetic acid, the solution was aged at 40°C for 4 hours and the reaction was then terminated. The crude solution was further washed in water at 30°C and low-boiling fractions were removed at 70°C/30 Torr to give 97.0 g of a product having the alicyclic epoxy compound (IC). With regard to the properties of the obtained product, the concentration of oxirane oxygen was 10.8%, the viscosity was 6, 700 cP (25°C), and the color hue (APHA) was 80. In ¹H-NMR, a peak around δ4.5 to 5 ppm that originated from the double bond almost disappeared and the formation of proton peak around δ2.9 to 3.3 ppm that originated from the epoxy group was observed. In this case, the concentration of high-molecular-weight components each having an elution time shorter than that of the alicyclic epoxy compound (IC) detected by a GPC analysis was 13.2% in terms of the area ratio, the concentration of impurities each having a retention time shorter than that of the alicyclic epoxy compound (IC) detected by a gas chromatography was 5.2% in terms of the area ratio, and the concentration of reactive intermediates was 2.2% in terms of the area ratio.

### [Comparative Example 4]

At 25°C, 167.7 g of an aqueous hydrogen peroxide having a concentration of 60%, 200 g of propionic acid, and 0. 45 g of sulfuric acid were mixed for 6 hours to synthesize perpropionic acid, which was in turn extracted with 700 g of benzene to give a benzene solution of the perpropionic acid having a concentration of 18.8% (water content: 0.41%). To the 3 L jacketed flask used in Example 1, 100 g of the 2,2-bis(cyclohexenyl) propane synthesized in Example 1 was placed. To this flask, 539.5 of the above-described benzene solution of the perpropionic acid was added dropwise over approximately 3 hours to become the temperature inside the reaction system to 40°C. After the completion of dropping, the solution was aged at 40°C for 4 hours and the reaction was then terminated. The crude solution was further washed in water at 40°C and low-boiling fractions were removed at 70°C/20 Torr to give 101.5 g of a product having the alicyclic epoxy compound (IA). With regard to the properties of the obtained product, the concentration of oxirane oxygen was 8.6%, the viscosity was 17,610 cP (25°C), and the color hue (APHA) was 180. In ¹H-NMR, a peak around δ4.5 to 5 ppm that originated from the inner double bond almost disappeared and the formation of proton peak around δ2.9 to 3.3 ppm that originated from the epoxy group was observed. In this case, the concentration of high-molecular-weight components each having an elution time shorter than that of the alicyclic epoxy compound (IA) detected by a GPC analysis was 36.9% in terms of the area ratio, the concentration of impurities each having a retention time shorter than that of the alicyclic epoxy compound (IA) detected by gas chromatography was 10.2% in terms of the area ratio, and the concentration of reactive intermediates was 3.5% in terms of the area ratio.

As described above, even having used the perpropionic acid containing water in small amounts, a product obtained has more inferior oxirane oxygen, color hue, and viscosity than those of a product obtained using the peracetic acid containing water in small amounts. This may be due to the effect of the hydrogen peroxide and the strong acid base catalyst that remain in the perpropionic acid.

### [Comparative Example 5]

At 30°C, 300 g of hydrogen peroxide having a concentration of 60% and 280 g of acetic acid were mixed for 3 hours to synthesize paracetic acid, which was in turn extracted with 1,000 g of ethyl acetate to give an ethyl acetate solution of the peracetic acid having a concentration of 21.8% (water content: 8.5%). To the 1 L jacketed flask used in Example 1, 100 g of the 2,2-bis(3',4'-cyclohexenyl) propane synthesized in Example 1 was placed. To this flask, 410.0g (water content: 8.5%) of the above-described ethyl acetate solution of the peracetic acid having a concentration of 21.8% was added dropwise over approximately 2 hours to become the temperature inside the reaction system to 30°C. After the completion of dropping, the solution was aged at 30°C for 4 hours and the reaction was then terminated. The crude solution was further washed in water at 20°C and low-boiling fractions were removed at 70°C/20 Torr to give 65.7 g of a product having the alicyclic epoxy compound (IA). With regard to the properties of the obtained product, the concentration of oxirane oxygen was 4.9%, the viscosity was 16,000 cP (25°C), and the color hue (APHA) was 260. In ¹H-NMR, a peak around δ4.5 to 5 ppm that originated from the inner double bond almost disappeared and the formation of proton peak around δ2. 9 to 3.3 ppm that originated from the epoxy group was observed. In this case, the concentration of high-molecular-weight components each having an elution time shorter than that of the alicyclic epoxy compound (IA) detected by a GPC analysis was 35.2% in terms of the area ratio, the concentration of impurities each having a retention time shorter than that of the alicyclic epoxy compound (IA) detected by a gas chromatography was 9.8% in terms of the area ratio, and the concentration of reactive intermediates was 3.2% in terms of the area ratio.

Hereinafter, there will be described Application Examples and Comparative Application Examples of the epoxy compound synthesized in Examples and Comparative Examples described above.

### 1. Example of curing with thermal cationic polymerization initiator

After the epoxy compound and a thermal cationic polymerization initiator were blended according to Table 1 (a value in the table represents part by weight, which is similarly given in the description below) , primary curing at 65°C for 2 hours and subsequent secondary curing at 150°C for 1 hour were carried out.

As a heat resistance index, temperatures at which loss modulus reached to 1GPa were compared, when glass-transition temperature (Tg) and dynamic viscoelasticity were measured with TMA.

Tg measurement: measured with TMA/SS6100 (manufactured by Seiko Instruments Inc.) based on JIS K7196.

Temperature at which loss modulus reaches 1GPa: measured with DMS 5200 (manufactured by Seiko Instruments Inc.).

Frequency for measurement: 10 Hz.

**Table 1**

| | Application Example 1 | Application Example 2 | Application Example 3 | Comparative Application Example 1 |
|---|---|---|---|---|
| Epoxy resin obtained in Example 1 | 100 | | | |
| Epoxy resin obtained in Example 2 | | 100 | | |
| Epoxy resin obtained in Example 3 | | | 100 | |
| Epoxy resin obtained in Comparative Example 1 | | | | 100 |
| SI-100L | 0.5 | 0.5 | 0.5 | 0.5 |
| T_{g}(°C) | not less than 300 | not less than 300 | not less than 300 | 198.6 |
| Temperature of 1GPa(°C) | not less than 300 | not less than 300 | not less than 300 | 273.0 |

| | | | | |
|---|---|---|---|---|
| SL-100L: Thermal cationic polymerization initiator (manufactured by Sanshin Chemical Industries, Co., Ltd.) | | | | |

### 2. Example of curing with an acid anhydride

After the epoxy compound, a curing agent, and a curing accelerator were blended according to Table 2, primary curing at 100°C for 2 hours and subsequent secondary curing at 150°C for 1 hour and at 180°C for 2 hours were carried out.

**Table 2**

| | Application Example 4 | Application Example 5 | Application Example 6 | Comparative Application Example 2 |
|---|---|---|---|---|
| Epoxy resin obtained in Example 1 | 100 | | | |
| Epoxy resin obtained in Example 3 | | 100 | | |
| Epoxy resin obtained in Comparative Example 1 | | | 100 | |
| YD-128 | | | | 100 |
| Rikacid MH-700 | 117.7 | 115.3 | 107.8 | 80.6 |
| BDMA | 1.2 | 1.1 | 1.0 | 0.8 |
| Tg(°C) | 245.9 | 241.4 | 227.4 | 128.8 |
| Appearance | Transparent | Transparent | Transparent | Yellowing |

| | | | | |
|---|---|---|---|---|
| YD-128: Bisphenol A-type epoxy resin (Manufactured by Toto Chemical Corporation, epoxy equivalent = 190, Viscosity = 13,600 mPa.s/25°C) Rikacid MH-700: methyl hexahydrophthalic anhydride (manufactured by New Japan Chemical, Co., Ltd.) BDMA: benzyldimethylamine (manufactured by Wako Pure Chemical Industries, Ltd.) | | | | |

### 3. Example of curing with photocationic curing catalyst

The epoxy compound and a curing catalyst were blended according to Table 3 and applied to an aluminum plate (A1050P) whose film thickness was brought up to 15 µm, followed by curing by irradiation with the dose of 175 mJ/cm².

The property of the film surface immediately after irradiation; the property of the film surface after 1 minute
o: Cured, Δ: Cured only in the surface × : Not cured with tack

Pencil hardness: evaluated according to JIS K5400.

**Table 3**

| | Applicatio n Example 7 | Application Example 8 | Application Example 9 | Comparative Application Example 3 | Comparative Application Example 4 |
|---|---|---|---|---|---|
| Epoxy resin obtained in Example 1 | 100 | | | | |
| Epoxy resin obtained in Example 3 | | 100 | | | |
| Epoxy resin obtained in Comparative Example 1 | | | 100 | | |
| CEL-2021P | | | | 100 | |
| YD-128 | | | | | 100 |
| UVACURE1591 | 3 | 3 | 3 | 3 | 3 |
| FC430 | 0.5 | 0.5 | 0.5 | 0.5 | 0.5 |
| Property of film surface immediately after irradiation | ο | ο | Δ | × | × |
| Property of film surface after one minute | o | o | o | o | × |
| Pencil hardness after 20 min. (scratch) | 2H | 2H | HB | HB | not cured |

| | | | | | |
|---|---|---|---|---|---|
| CEL-2021P: 3,4-epoxycyclohexenylmethyl-3',4'-epolxycyclohexyenyl carboxylate manufactured by Daicel Chemical Industries, Ltd. YD-128: Bisphenol A-type epoxy resin (Manufactured by Toto Chemical Corporation, epoxy equivalent = 190, Viscosity = 13, 600 mPa·s/25°C) UVACURE 1591: sulfoniuim Salt-based photo cationic polymerization initiator, manufactured by Daicel UCB, Industries, Ltd. FC430: Leveling agent, manufactured by 3M, CO., Ltd. | | | | | |

### Industrial Applicability

The applications of a high-purity alicyclic epoxy compound of the present invention is exemplified by transparent materials for encapsulation, transparent films, transparent sheets, insulating materials between layers, acid scavengers, furniture coatings, decorative coatings, coatings for beverage cans and the other cans, adhesives, undercoatings for automobiles, sealers, finishings, inks for textual information or image information, sealants for electronic parts, photoresists suitable for manufacturing printing plates or printing circuit boards, casting print rolls, formulations for molded materials or molded products made of formulations for forming sheets, solvents, flame retardants, intermediates for producing other compounds useful in various final uses including pharmaceuticals and medical implements. Particularly, a cured product that is cured using this high-purity alicyclic epoxy compound is significantly enhanced especially in transparency and heat resistance and can be employed in various applications such as transparent materials for encapsulation.

## Claims

1. A high-purity alicyclic epoxy compound represented by the following general formula (I): 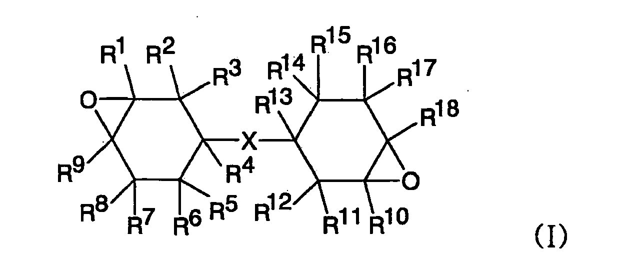 (wherein X is a divalent group selected from the group consisting of an oxygen atom, a sulfur atom, -SO-, -SO₂-, -CH₂-, -C(CH₃)₂-, -CBr₂-, -C(CBr₃)₂-, and -C(CF₃)₂-; R¹ to R¹⁸ each may be identical or different from each other and are a hydrogen atom, a halogen atom, a hydrocarbon group that may contain an oxygen atom or halogen atom, or an alkoxy group that may have a substituent),
in which the concentration of high-molecular-weight components having an elution time shorter than that of the alicyclic epoxy compound represented by the general formula-(I) in detection by a gel permeation chromatography (hereinafter, GPC) is 5.5% or less with respect to the sum total of all of detected peak areas in terms of the peak area ratio per elution time.

2. The high-purity alicyclic epoxy compound according to claim 1,
in which the concentration of impurities having a retention time shorter than that of the alicyclic epoxy compound represented by the above general formula (I) in detection by gas chromatography is 19.5% or less with respect to the sum total of all of detected peak areas in terms of the peak area ratio per retention time.

3. The high-purity alicyclic epoxy compound according to claim 1 or claim 2,
in which the concentration of reactive intermediate compounds represented by the following general formula (III): 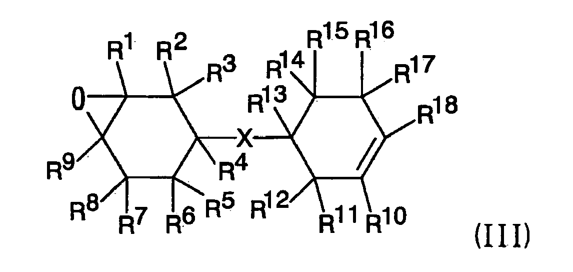 (wherein X is a divalent group selected from the group consisting of an oxygen atom, a sulfur atom, -SO-, -SO₂-, -CH₂-, -C(CH₃)₂-, -CBr₂-, -C (CBr₃)₂-, and -C(CF₃)₂-; R¹ to R¹⁸ each may be identical or different from each other and are a hydrogen atom, a halogen atom, a hydrocarbon group that may contain an oxygen atom or halogen atom, or an alkoxy group that may have a substituent),
in detection by gas chromatography is 4.5% or less with respect to the sum total of all of detected peak areas in terms of the peak area ratio per retention time.

4. The high-purity alicyclic epoxy compound according to any of claims 1 to 3, wherein a color hue (APHA) is 60 or less.

5. The high-purity alicyclic epoxy compound according to any one of claims 1 to 4, wherein the alicyclic epoxy compound is produced by epoxidizing, with an aliphatic percarboxylic acid having substantially no water, an alicyclic olefin compound represented by the following general formula (II): 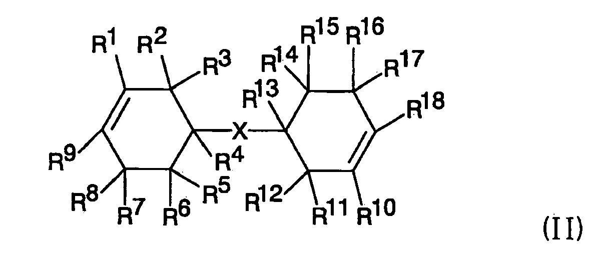 (wherein X is a divalent group selected from the group consisting of an oxygen atom, a sulfur atom, -SO-, -SO₂-, -CH₂-, -C(CH₃)₂-, -CBr₂-, -C(CBr₃)₂-, and -C (CF₃) ₂-; R¹ to R¹⁸ each may be identical or different from each other and are a hydrogen atom, a halogen atom, a hydrocarbon group that may contain an oxygen atom or halogen atom, or an alkoxy group that may have a substituent),
which is 95.0% or more with respect to the sum total of all of detected peak areas in terms of the peak area ratio determined by a gas chromatography.

6. The high-purity alicyclic epoxy compound according to claim 5, wherein the alicyclic epoxy compound is obtained by epoxidation followed by the removal of a solvent and purification by distillation.

7. A process for the production of a high-purity alicyclic epoxy compound,
in which an alicyclic olefin compound represented by the following general formula (II) 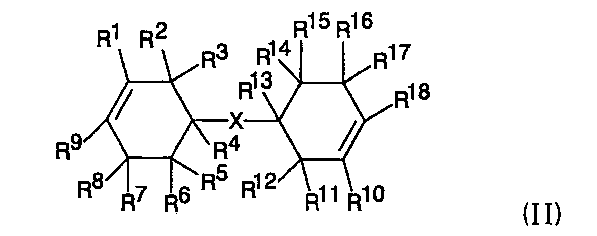 is epoxidized with an aliphatic percarboxylic acid having substantially no water followed by the removal of a solvent to produce an alicyclic epoxy compound represented by the general formula (I) 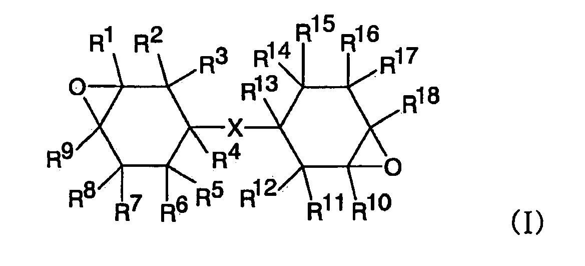 (in the formulas (I) and (II), X is a divalent group selected from the group consisting of an oxygen atom, a sulfur atom, -SO-, -SO₂-, -CH₂-, -C(CH₃)₂-, -CBr₂-, -C(CBr₃)₂-, and -C(CF₃)₂-; R¹ to R¹⁸ each may be identical or different from each other and are a hydrogen atom, a halogen atom, a hydrocarbon group that may contain an oxygen atom or halogen atom, or an alkoxy group that may have a substituent),
that is in turn subjected to purification by distillation to thereby the high-purity alicyclic epoxy compound wherein the concentration of high-molecular-weight components having an elution time shorter than that of the alicyclic epoxy compound in detection by GPC analysis is 5.5% or less with respect to the sum total of all of detected peak areas in terms of the peak area ratio per elution time.

8. The process for the production of a high-purity alicyclic epoxy compound according to claim 7,
in which the concentration of impurities having a retention time shorter than that of the alicyclic epoxy compound represented by the above general formula (I) in detection by gas chromatography is 19.5% or less with respect to the sum total of all of detected peak areas in terms of the peak area ratio per retention time.

9. The process for the production of a high-purity alicyclic epoxy compound according to claim 7 or claim 8,
the concentration of reactive intermediate compounds represented by the above general formula (III) in detection by gas chromatography is 4.5% or less with respect to the sum total of all of detected peak areas in terms of the peak area ratio per retention time.

10. The process for the production of a high-purity alicyclic epoxy compound according to any one of claims 7 wherein a color hue (APHA) is 60 or less.

11. The process for the production of a high-purity alicyclic epoxy compound according to any one of claims 7 to 10, wherein the aliphatic percarboxylic acid is obtained by the oxidation of a corresponding aldehyde.

12. The process for the production of a high-purity alicyclic epoxy compound according to any one of claims 7 to 11, wherein a water content in the aliphatic percarboxylic acid is 0.8% by weight or less.

13. The process for the production of a high-purity alicyclic epoxy compound according to any one of claims 7 to 12, wherein the aliphatic percarboxylic acid is a peracetic acid.

14. The process for the production of a high-purity alicyclic epoxy compound according to any one of claims 7 to 13, wherein the purification by distillation is carried out at a heating temperature ranging of 100 to 350°C and at a pressure of 50 to 0.01 Torr.

15. The process for the production of a high-purity alicyclic epoxy compound according to any one of claims 8 to 14, wherein the aliphatic percarboxylic acid is an ethyl acetate solution.

16. A photo-curable and/or heat-curable epoxy resin composition comprising the epoxy compound according to any one of claims 1 to 7; an epoxy group-containing compound optionally added; and a curing agent or a curing catalyst.

17. A cured product that is obtained by curing the curable epoxy resin composition according to claim 16.

18. A transparent material for encapsulation made of the high-purity alicyclic epoxy compound according to any one of claims 1 to 7.

19. An adhesive made of the high-purity alicyclic epoxy compound according to any one of claims 1 to 7.

20. The cured product according to claim 18, wherein the cured product is at least one selected from a transparent film, transparent sheet, an insulating material between layers, a coated film, and a paint film.
